Europäisches Patentamt

European Patent Office

Office européen des brevets

(11) Publication number: **0 510 575 A1**

# EUROPEAN PATENT APPLICATION

(21) Application number: **92106788.0**

(22) Date of filing: **21.04.92**

(51) Int. Cl.⁵: **A61D 1/02, A61M 5/32**

(30) Priority: **22.04.91 US 689414**

(43) Date of publication of application:
**28.10.92 Bulletin 92/44**

(84) Designated Contracting States:
**PT**

(71) Applicant: **SCHERING CORPORATION**
**2000 Galloping Hill Road**
**Kenilworth New Jersey 07033(US)**

(72) Inventor: **Liebowitz, Stephen M.**
**70 Beechwood Circle**
**Neshanic Station, New Jersey 08853(US)**

(74) Representative: **von Kreisler, Alek,**
**Dipl.-Chem. et al**
**Patentanwälte Von Kreisler-Selting-Werner,**
**Deichmannhaus am Hauptbahnhof**
**W-5000 Köln 1(DE)**

(54) Mastitis infusion system and frangible mastitis cannula closure therefor.

(57) A mastitis cannula closure (10) for a mastitis infusion system of the type including a syringe barrel (16), a cannula (12) having a free tip end (12a) with an opening (30) thereat, and a connecting assembly which connects the cannula to the syringe barrel, the mastitis cannula closure including a cap-like member having a proximal end (32) and a distal end (36), with a portion (34) of the cap-like member being substantially rigid; a restriction for restricting passage (36) of the free tip end of the cannula out of the cap-like member, the restriction being connected with the distal end of the cap-like member; a mounting device (32) for removably holding the cap-like member in surrounding relation to the cannula at a first position at which the cap-like member is in covering relation to the free tip end of the cannula, the mounting device permitting removal of the cap-like member from the surrounding relation to expose at least substantially the entire length of the cannula; and the substantially rigid portion of the cap-like member being movable by an external force from the first position to a second position at which the free tip end (12a) extends through the restriction and only partially out of the cap-like member, and the substantially rigid portion being maintained at the second position after removal of the external force.

FIG.2

## BACKGROUND OF THE INVENTION

The present invention relates generally to the administration of a veterinary pharmacological composition and, more particularly, is directed to a frangible mastitis cannula closure for an applicator used to administer the pharmacological composition.

Mastitis is a disease that results in inflammation of the udder of a cow, caused by infection. As a result of this mastitis or inflammation, the milk production by the cow is substantially reduced, thereby detrimentally affecting profitability.

In order to treat this disease, veterinary pharmacological compositions, such as anti-infectives or antibiotics, are generally administered by injecting the same into the cow's udder through the teat canal by a cannula and infusion syringe assembly. The cannula which extends through the teat into the udder is generally made from a smooth plastic material to prevent damage or irritation to the animal's tissue.

In order to prevent contamination of the pharmacological composition and/or leakage thereof, it has been customary to use a single piece plastic, slip-type cap which snap-fittingly or frictionally engages the outer surface of the cannula or the hub of the syringe at the base of the cannula. In use, after the cap has been fully removed from the cannula, the cannula is inserted into the cow's teat, passed through the teat canal and positioned within the teat cistern. Thereafter, the syringe is activated to inject the pharmacological composition directly into the cow's teat cistern. In such case, substantially the entire length of the cannula is inserted into the cow's teat canal.

Alternatively, in some situations, injection may occur directly into the cow's udder in order to kill any bacteria in the teat canal itself. In such case, in addition to, or as an alternative, only a small tip portion of the cannula is inserted into the teat canal for a depth of approximately 3 mm.

However, conventional cannula and insertion syringe assemblies are not adapted to be used quickly and easily at different depths in the cow's udder.

Accordingly, there has been developed a system which utilizes a two-part cap or closure for the cannula so as to permit insertion at two different depths. Specifically, in U.S. Patent No. 4,850,970 to Sutherland, a two-part mastitis cannula cap is disclosed in which an inner cap is snap-fittingly inserted over the cannula, the inner cap having an opening at the distal end thereof through which the tip of the cannula extends for use in the alternative procedure in which only the tip end of the cannula is inserted into the teat canal. The assembly further includes an outer cap which snap- fittingly is received over the inner cap and which covers the free distal end of the cannula. If it is desired to insert only the tip of the cannula into the teat canal, only the outer cap is removed, thereby exposing only the tip end of the cannula. If, on the other hand, the entire cannula is to be inserted into the cow's udder, the entire inner cap and outer cap assembly is removed from the cannula. A device of substantially identical construction is described in PCT Published Application No. WO 90/07913, published on July 26, 1990.

However, because a two-cap system is used, the two-cap system adds additional complexity and cost to the applicator.

Other patents of a related interest are as follows:

U.S. Patent No. 4,892,521 to Laico et al. discloses a protective cover assembly for a hypodermic needle in which the distal end of the cap has an interiorly projecting, internal protective flap which covers a central axial aperture that is dimensioned to accept the needle therethrough in the retracted mode. The needle base includes a slotted wing with diametrically spaced apertures therein which accept guide rods as part of the protective cover assembly and which guide the cap of the protective cover assembly to the protective position and retain the cap to the syringe. However, this assembly requires slotted wings and guide rods which further complicates the structure of the assembly. Also, the cover cannot be completely removed to allow complete insertion of the needle.

Figs. 5-7 of this patent show a second embodiment of a protective cap assembly that includes three telescoping shield sections which can be compacted in a nested configuration, allowing the needle to be exposed for usage. See also U.S. Patent No. 4,897,083 to Martell for a similar disclosure.

Finally, a third embodiment of the patent is shown in Figs. 8 and 9 in which the sheath for the needle is corrugated so as to be collapsible. However, this embodiment also requires guide rods externally of the corrugated sheath.

U.S. Patent No. 4,968,304 to Alter et al. discloses a syringe which is mounted to a movable wall slidably retained within a hollow body having an opening at the opposite end thereof. The sheath or hollow body is moved rearwardly of the needle so as to expose the same. However, there is no seal at the distal end of the sheath that is pierced by the needle, but rather, a removable cap is provided thereat.

U.S. Patent No. 4,950,250 to Haber et al. discloses a collapsible needle cover. However, with this patent, hinges are provided, which render the device relatively complicated and unfeasible from a commercial standpoint.

U.S. Patent No. 4,915,697 to DuPont discloses a hypodermic needle assembly including a sleeve which telescopically fits over the needle and includes an axially rigid upper mounting portion which mounts over the needle base. The sleeve includes an axially collapsible intermediate portion and an axially rigid lower portion defining an axially extending central bore for receiving the needle therein. A sheath is designed to fit telescopically over the sleeve and has a length generally corresponding to the length of the sleeve. to use the same, the sheath is removed and a downward pressure is exerted on the syringe so that the intermediate portion of the sleeve collapses axially as the pointed tip of the needle shaft enters the subject, to selectively expose the needle. Following withdrawal, the sleeve selectively expands axially to cover the needle tip as it emerges from the subject. However, to expose the needle requires a constant pressure on the sleeve, which renders the same impractical as a mastitis cannula closure.

Finally, U.S. Patent No. 4,664,653 to Sagstetter et al. discloses a manually operated, reusable injection apparatus including a rubber sheath surrounding the needle and which is collapsible during insertion of the needle.

## OBJECTS AND SUMMARY OF THE INVENTION

Accordingly, it is an object of the present invention to provide a mastitis infusion system and a mastitis cannula closure therefor that overcomes the aforementioned problems with the prior art.

It is another object of the present invention to provide a mastitis cannula closure of a single piece construction which can selectively control the depth of insertion of the cannula into the udder of a cow.

It is still another object of the present invention to provide a mastitis cannula closure which is relatively inexpensive and easy to manufacture and use.

In accordance with an aspect of the present invention, a mastitis cannula closure for a mastitis infusion system of the type including a syringe barrel, a cannula having a free tip end with an opening thereat, and a connecting assembly which connects the cannula to the syringe barrel, includes a cap-like member having a proximal end and a distal end, with a portion of the cap-like member being substantially rigid; restriction means for restricting passage of the free tip end of the cannula out of the cap-like member, the restriction means being connected with the distal end of the cap-like member; mounting means for removably holding the cap-like member in surrounding relation to the cannula at a first position at which the cap-like member is in covering relation to the free tip end of the cannula, the mounting means permitting removal of the cap-like member from the surrounding relation to expose at least substantially the entire length of the cannula; and the substantially rigid portion of the cap-like member being movable by an external force from the first position to a second position at which the free tip end extends through the restriction means and only partially out of the cap-like member, and the substantially rigid portion being maintained at the second position after removal of the external force.

In accordance with another aspect of the present invention, a mastitis cannula closure for a mastitis infusion system of the type including a syringe barrel, a cannula having a free tip end with an opening thereat, and a connecting assembly which connects the cannula to the syringe barrel, includes a cap-like member having a proximal end and a distal end, the cap-like member including a substantially rigid portion and a collapsible portion connected with the substantially rigid portion; restriction means for restricting passage of the free tip end of the cannula out of the cap-like member, the restriction means being connected with the distal end of the cap-like member; mounting means for removably holding the cap-like member in surrounding relation to the cannula at a first position at which the cap-like member is in covering relation to the free tip end of the cannula, the mounting means permitting removal of the cap-like member from the surrounding relation to expose at least substantially the entire length of the cannula; and the collapsible portion being collapsible in an axial direction of the cannula by an external axial force so as to move the substantially rigid portion of the cap-like member from the first position to a second position at which the free tip end extends through the restriction means and only partially out of the cap-like member, and the collapsible portion being maintained in the collapsed condition and the substantially rigid portion being maintained at the second position after removal of the external force.

In accordance with still another aspect of the present invention, a mastitis cannula closure for a mastitis infusion system of the type including a syringe barrel, a cannula having a free tip end with an opening thereat, and a connecting assembly which connects the cannula to the syringe barrel, includes a substantially rigid cap-like member having a proximal end and a distal end; restriction means for restricting passage of the free tip end of the cannula out of the cap-like member, the restriction means being connected with the distal end of the cap-like member; mounting means for removably holding the cap-like member in surrounding relation to the cannula at a first position at which the cap-like member is in covering relation to the free tip end of the cannula, the mounting means permitting removal of the cap-like member from the surrounding relation to expose at least substantially the entire length of the cannula; and the substantially rigid portion of the cap-like member being movable by an external force from the first position to a second position at which the mounting means removably holds the cap-like member in surrounding relation to the cannula and at which the free tip end extends through the restriction means and only partially out of the cap-like member, and the substantially rigid portion being maintained at the second position after removal of the external force.

In accordance with yet another aspect of the present invention, a mastitis infusion system includes a syringe barrel; a cannula having a free tip end with an opening thereat; a connecting assembly which connects the cannula to the syringe barrel; and mastitis cannula closure means for covering the cannula, the mastitis cannula closure means including a substantially rigid cap-like member having a proximal end and a distal end, restriction means for restricting passage of the free tip end of the cannula out of the cap-like member, the restriction means being connected with the distal end of the cap-like member, mounting means for removably holding the cap-like member in surrounding relation to the cannula at a first position at which the cap-like member is in covering relation to the free tip end of the cannula, the mounting means permitting removal of the cap-like member from the surrounding relation to expose at least substantially the entire length of the cannula, and the substantially rigid portion of the cap-like member being movable by an external force from the first position to a second position at which the mounting means removably holds the cap-like member in surrounding relation to the cannula and at which the free tip end extends through the restriction means and only partially out of the cap-like member, and the substantially rigid portion being maintained at the second position after removal of the external force.

In accordance with a further aspect of the present invention, a mastitis infusion system includes a syringe barrel; a cannula having a free tip end with an opening thereat; a connecting assembly which connects the cannula to the syringe barrel; and mastitis cannula closure means for covering the cannula, the mastitis cannula closure means including a cap-like member having a proximal end and a distal end, the cap-like member including a substantially rigid portion and a collapsible portion connected with the substantially rigid portion, restriction means for restricting passage of the free tip end of the cannula out of the cap-like member, the restriction means being connected with the distal end of the cap-like member, mounting means for removably holding the cap-like member in surrounding relation to the cannula at a first position at which the cap-like member is in covering relation to the free tip end of the cannula, the mounting means permitting removal of the cap-like member from the surrounding relation to expose at least substantially the entire length of the cannula, and the collapsible portion being collapsible in an axial direction of the cannula by an external axial force so as to move the substantially rigid portion of the cap-like member from the first position to a second position at which the free tip end extends through the restriction means and only partially out of the cap-like member, and the collapsible portion being maintained in the collapsed condition and the substantially rigid portion being maintained at the second position after removal of the external force.

The above and other objects, features and advantages of the present invention will become readily apparent from the following detailed description thereof which is to be read in connection with the accompanying drawings.

BRIEF DESCRIPTION OF THE DRAWINGS

Fig. 1 is a cross-sectional view of a mastitis cannula closure according to one embodiment of the present invention, showing the same assembled with a mastitis cannula;

Fig. 2 is a cross-sectional view similar to Fig. 1, showing the mastitis cannula closure in its collapsed position with the distal tip end of the cannula rupturing and extending through the closed end of the closure;

Fig. 3 is a cross-sectional view of a mastitis cannula closure according to another embodiment of the present invention;

Fig. 4 is an enlarged elevational view of a portion of the mastitis cannula closure of Fig. 3, viewed from the right side thereof;

Fig. 5 is a cross-sectional view of the mastitis cannula closure of Fig. 4, taken along line 5-5 thereof;

Fig. 6 is a cross-sectional view of the mastitis cannula closure of Fig. 3, at a first position on the cannula at which the closed end is in covering relation to the distal tip end of the cannula; and

Fig. 7 is a cross-sectional view of the mastitis cannula closure of Fig. 6, showing the same at a second position on the cannula at which the distal tip end ruptures the closed end and extends partially therethrough.

DETAILED DESCRIPTION OF THE PREFERRED EMBODIMENTS

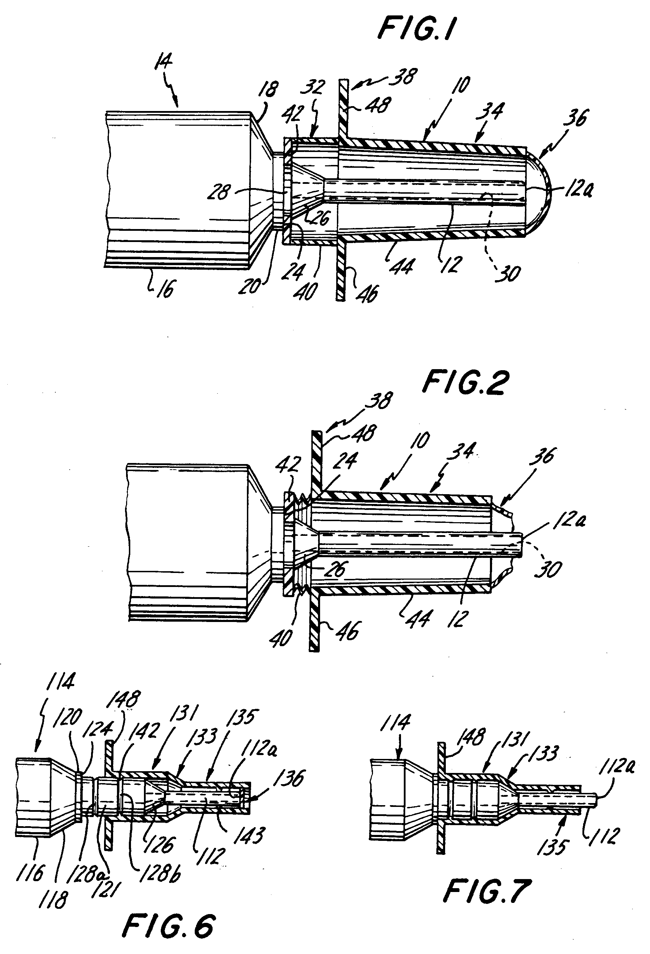

Referring to the drawings in detail, and initially to Figs. 1 and 2 thereof, a mastitis cannula closure 10 according to one embodiment of the present invention, is provided in covering relation to a cannula 12 of a mastitis infusion system 14 of the type generally described in U.S. Patent No. 4,850,970 to Sutherland and published PCT Application No. WO 90/07913, published on July 26, 1990, the entire disclosures of which are incorporated herein by reference.

Specifically, mastitis infusion system 14 generally includes a hypodermic syringe barrel 16 which is generally made from a sterile and disposable plastic material, such as a low density polyethylene. The distal end 18 of syringe barrel 16 is frusto-conically tapered, terminating in an annular neck 20 of a fixed diameter. A reduced diameter neck 22 is centrally formed at the distal end of annular neck 20 and has a fixed diameter less than that of annular neck 20. Accordingly, an annular front face 24 is formed as the distal face of annular neck 20 in surrounding relation to reduced diameter neck 22. A frusto-conical hub 26 has its greater diameter end mounted to reduced diameter neck 22 in spaced relation from front face 24. Because the greater diameter end of frusto-conical hub 26 has a diameter greater than that of reduced diameter neck 22, as shown in the figures, an annular groove 28 is formed between the greater diameter end of frusto-conical hub 26 and annular front face 24 of annular neck 20.

The proximal end of cannula 12 is of the same diameter as the smaller diameter end of frusto-conical hub 26 and is secured therewith. It will be appreciated that hypodermic syringe barrel 16, distal end 18, annular neck 20, reduced diameter neck 22, hub 26 and cannula 12 are all provided with a central or axial opening 30 therethrough to permit the supply of the veterinary pharmacological composition from syringe barrel 16 to the distal or free end 12a of cannula 12, which is open.

Cannula 12 should be sufficiently long to provide deep penetration into the udder of the cow so as to penetrate into the cow's feat cistern. In this regard, although not necessary, it is preferable that cannula 12 be slightly tapered so as to facilitate insertion and removal thereof. As an example, cannula 22 can be 20 to 25 mm in length, although the size of mastitis infusion system 14, including the overall length of cannula 12 can be modified. Preferably, mastitis infusion system 14, including hypodermic syringe barrel 16, distal end 18, annular neck 20, reduced diameter neck 22, frusto-conical hub 26 and cannula 14 are constructed of a one-piece molded plastic material, such as a low-density, polyethylene material.

In accordance with the present invention, mastitis cannula closure 10 is formed by a collapsible section 32 removably mounted on mastitis infusion system 14, a rigid cover section 34 in covering relation to a substantial portion of cannula 12 and integrally formed with the distal end of collapsible section 32, a frangible seal section 36 secured to the distal end of rigid cover section 34, and engagement means 38 secured to rigid cover section 34 for permitting movement of rigid cover section 34 between a first position (Fig. 1) in covering relation to cannula 12 at which frangible seal section 36 is in covering relation to the open free tip end 12a of cannula 12, and a second position (Fig. 2) in partial covering relation to cannula 12 at which open free tip end 12a of cannula 12 ruptures frangible seal section 36 and extends partially therethrough a small distance, for example, 3 mm.

Specifically, collapsible section 32 includes an annular side wall 40 of a sufficiently small thickness such that an axial force thereon will cause annular side wall 40 to collapse in a corrugated or accordion-like fashion, as shown in Fig. 2. Side wall 40 may also be scored or formed in an already pleated manner to aid such collapsing. The proximal end of annular side wall 40 is formed with an annular inturned lip 42 which seats within annular groove 28 defined between frusto-conical hub 26 and front face 24, in a snap-fitting manner so as to releasably hold mastitis cannula closure 10 on mastitis infusion system 14. It will be appreciated that collapsible section 32 is thereby in covering relation to frusto-conical hub 26 and the proximal end of cannula 12.

Rigid cover section 34 is formed by an annular side wall 44 which may have a slightly tapered configuration, as shown in the figures, and which is connected to the distal end of annular side wall 40 of collapsible section 32, as an axial extension thereof. Preferably, annular side wall 44 has its distal or free end extending at least as far as the distal or free tip end 12a of cannula 12 at the distal end thereof. However, annular side wall 44 has a sufficient thickness, and is made of a sufficiently durable plastic material, so as to prevent collapsing thereof during normal use, thereby differing from the reduced thickness annular side wall 40 of collapsible section 32.

The distal end of annular side wall 44 is closed by frangible seal section 36 which can be made of a reduced thickness plastic, a foil or the like. Although frangible seal section 36 is shown in Fig. 1 in a dome-like configuration, frangible seal section 36 can be planar or have any other suitable configuration.

Finally, engagement means 38 of mastitis cannula closure 10 includes diametrically opposite finger wings 46 and 48 extending radially outward from and connected with the outer surface of annular side wall 44 of rigid cover section 34 at the proximal end thereof. Alternatively, finger wings 46 and 48 can be replaced by an annular flange or the like.

In operation, when it is desired to insert the cannula fully into the cow's teat so that it passes through the teat canal and into the teat cistern, mastitis cannula closure 10 must be fully removed. Thus, in the same manner as with U.S. Patent No. 4,850,970 to Sutherland and published PCT Application No. WO 90/07913, annular interned lip 42 is removed from annular groove 28 and pulled forward or distally so as to remove mastitis cannula closure 10 from mastitis infusion system 14, thereby exposing the entire length of cannula 12. In such case, substantially the entire length of cannula 12 is inserted into the cow's teat canal.

However, as earlier described, in some situations, injection may occur directly into the cow's udder in order to kill any bacteria in the teat canal itself. In such case, it is only necessary to insert a small tip portion of free tip end 12a of cannula 12 into the teat canal for a depth of approximately 3 mm.

With the present invention, and without removal of mastitis cannula closure 10 from mastitis infusion system 14, the person merely grasps finger wings 46 and 48 and moves the same proximally or to the left of Fig. 1, to the position shown in Fig. 2. As a result, annular side wall 40 collapses in an accordion fashion, as shown in Fig. 2, because of the reduced thickness thereof. Because finger wings 46 and 48 are integrally formed with rigid annular side wall 44 of rigid cover section 34, rigid cover section 34 also moves proximally. Accordingly, the free tip end 12a of cannula 12 pierces frangible seal section 36 so as to expose only a portion thereof, for example, 3 mm thereof.

It will therefore be appreciated that, with the present invention, a single piece mastitis cannula closure 10 is provided which can be used to expose the entire cannula 12 by removal of closure 10, or alternatively, by collapsing collapsible section 32 in order to expose only free tip end 12a thereof.

Because of the single piece arrangement and the manner of operation, there is no need for both an inner and outer cap as with U.S. Patent No. 4,850,970 or published PCT Application No. WO 90/07913.

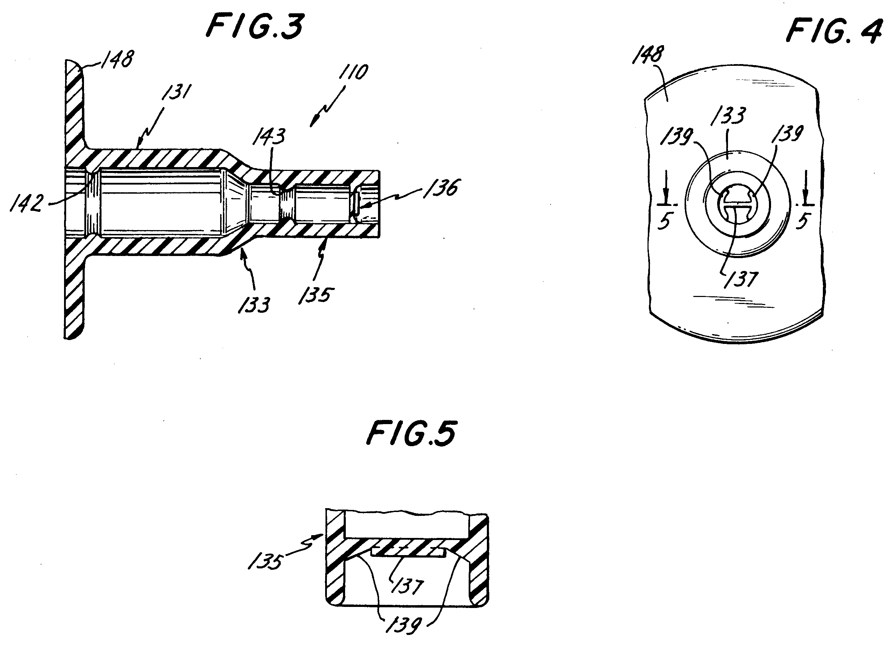

Referring now to Figs. 3-7, a mastitis cannula closure 110 according to another embodiment of the present invention will now be described in which elements corresponding to those of mastitis cannula closure 10 are identified by the same reference numerals augmented by 100, and a detailed description thereof will be omitted herein for the sake of brevity.

As shown therein, mastitis infusion system 114 includes the same hypodermic syringe barrel 116, frusto-conical distal end 118 and annular neck 120 of a fixed diameter. From there, however, mastitis infusion system 114 differs from mastitis infusion system 14. Specifically, a hollow cylindrical section 121 extends axially from annular neck 120 and has a smaller diameter than annular neck 120 so as to produce the same annular front face 124. Cylindrical section 121 is provided with two axially spaced annular grooves 128a and 128b, annular groove 128b being positioned distally with respect to annular groove 128a. From the distal end of cylindrical section 121, mastitis infusion system 114 includes the same frusto-conical hub 126 with the greater diameter end thereof having a diameter equal to that of cylindrical section 121 and connected therewith, and with cannula 112 extending from the lesser diameter end of hub 126 and in axial alignment therewith.

In accordance with the second embodiment of the present invention, mastitis cannula closure 110 is made entirely of a rigid plastic material, and particularly, includes a proximal cylindrical section 131 connected by a substantially frusto-conical connecting section 133 to a distal cylindrical section 135, with frusto-conical connecting section 133 being inclined at an angle similar to the inclination of frusto-conical hub 126. The inner diameter of distal cylindrical section 135 is less than that of proximal cylindrical section 131, but is still greater than the outer diameter of cannula 12. Further, an annular flange 148 extends radially outward from the proximal end of proximal cylindrical section 131. In this manner, by grasping annular flange 148, mastitis cannula closure 110 can be moved from the position shown in Fig. 6 to the position shown in Fig. 7.

In this regard, an inner annular bead 142 is formed along the inner wall of proximal cylindrical section 131 near the proximal end thereof, as best shown in Fig. 3. When mastitis cannula cover 110 is in the position of Fig. 6, bead 142 engages within annular groove 128b so as to removably secure mastitis cannula closure 110 on cylindrical section 121 at the position of Fig. 6. On the other hand, annular flange 148 can be gripped to move mastitis cannula 110 to the left of Fig. 6, that is, to the position of Fig. 7. In such case, bead 142 engages within annular groove 128a to removably secure mastitis cannula closure 110 in the position of Fig. 7. In such position, it will be appreciated that the proximal face of annular flange 148 engages against annular front face 124 of annular neck 120 to prevent bead 142 from travelling past annular groove 128a and to define the extent that free tip end 112a of cannula 112 extends from mastitis cannula cover 110. Further, in the position of Fig. 7, because the inclinations of frusto-conical connecting section 133 and frusto-conical hub 126 are substantially identical, frusto-conical connecting section 133 seats snugly against frusto-conical hub 126.

In addition, mastitis cannula closure 110 includes an annular sealing bead 143 along the inner wall of distal cylindrical section 135, with annular sealing bead 143 engaging the outer surface of cannula 112 so as to provide a seal thereat.

As shown best in Figs. 3-5, a rupturable seal section 136 is provided internally at the distal end of distal cylindrical section 135. Specifically, a reinforced strip 137 extends diametrically across the inner bore of distal cylindrical section 135 and is integrally formed at opposite ends with the inner walls of distal cylindrical section 135. The outer ends of reinforced strip 137 are further reinforced by arcuate reinforcing strips 139 that extend partially around the inner wall of distal cylindrical section 135.

With this arrangement, when mastitis cannula closure 110 is moved from the position of Fig. 6 to the position of Fig. 7, the free tip end 112a thereof ruptures diametrically reinforced strip 137 so to extend partially therethrough. In order to utilize mastitis infusion system 114 so as to insert the entire cannula 112 within the teat canal, mastitis cannula closure 110 is removed in its entirety.

As with mastitis cannula closure 10, mastitis cannula closure 110 is constructed as a single piece closure, contrary to the arrangement of U.S. Patent No. 4,850,970.

Having described specific preferred embodiments of the invention with reference to the accompanying drawings, it will be appreciated that the present invention is not limited to those precise embodiments, and that various changes and modifications can be effected therein by one of ordinary skill in the art without departing from the spirit or scope of the invention as defined by the appended claims.

**Claims**

1.  A mastitis cannula closure for a mastitis infusion system of the type including a syringe barrel, a cannula having a free tip end with an opening thereat, and a connecting assembly which connects the cannula to the syringe barrel, said mastitis cannula closure comprising:
    a) a cap-like member having a proximal end and a distal end, with a portion of said cap-like member being substantially rigid;
    b) restriction means for restricting passage of the free tip end of said cannula out of said cap-like member, said restriction means being connected with the distal end of said cap-like member;
    c) mounting means for removably holding said cap-like member in surrounding relation to said cannula at a first position at which said cap-like member is in covering relation to the free tip end of said cannula, said mounting means permitting removal of said cap-like member from said surrounding relation to expose at least substantially the entire length of said cannula; and
    d) said substantially rigid portion of said cap-like member being movable by an external force from said first position to a second position at which said free tip end extends through said restriction means and only partially out of said cap-like member, and said substantially rigid portion being maintained at said second position after removal of said external force.

2.  A mastitis cannula closure according to Claim 1, wherein said restriction means includes rupturable means for permitting passage of the free tip end of said cannula out of said cap-like member only upon rupture thereof by said cannula.

3.  A mastitis cannula closure for a mastitis infusion system of the type including a syringe barrel, a cannula having a free tip end with an opening thereat, and a connecting assembly which connects the cannula to the syringe barrel, said mastitis cannula closure comprising:
    a) a cap-like member having a proximal end and a distal end, said cap-like member including a substantially rigid portion and a collapsible portion connected with said substantially rigid portion;
    b) restriction means for restricting passage of the free tip end of said cannula out of said cap-like member, said restriction means being connected with the distal end of said cap-like member;
    c) mounting means for removably holding said cap-like member in surrounding relation to said cannula at a first position at which said cap-like member is in covering relation to the free tip end of said cannula, said mounting means permitting removal of said cap-like member from said surrounding relation to expose at least substantially the entire length of said cannula; and
    d) said collapsible portion being collapsible in an axial direction of said cannula by an external axial force so as to move said substantially rigid portion of said cap-like member from said first position to a second position at which said free tip end extends through said restriction means and only partially out of said cap-like member, and said collapsible portion being maintained in said collapsed condition and said substantially rigid portion being maintained at said second position after removal of said external force.

4. A mastitis cannula closure according to Claim 3, wherein said restriction means includes rupturable means for permitting passage of the free tip end of said cannula out of said cap-like member only upon rupture thereof by said cannula.

5. A mastitis cannula closure for a mastitis infusion system of the type including a syringe barrel, a cannula having a free tip end with an opening thereat, and a connecting assembly which connects the cannula to the syringe barrel, said mastitis cannula closure comprising:
a) a substantially rigid cap-like member having a proximal end and a distal end;
b) restriction means for restricting passage of the free tip end of said cannula out of said cap-like member, said restriction means being connected with the distal end of said cap-like member;
c) mounting means for removably holding said cap-like member in surrounding relation to said cannula at a first position at which said cap-like member is in covering relation to the free tip end of said cannula, said mounting means permitting removal of said cap-like member from said surrounding relation to expose at least substantially the entire length of said cannula; and
d) said substantially rigid portion of said cap-like member being movable by an external force from said first position to a second position at which said mounting means removably holds said cap-like member in surrounding relation to said cannula and at which said free tip end extends through said restriction means and only partially out of said cap-like member, and said substantially rigid portion being maintained at said second position after removal of said external force.

6. A mastitis cannula closure according to Claim 5, wherein said restriction means includes rupturable means for permitting passage of the free tip end of said cannula out of said cap-like member only upon rupture thereof by said cannula.

7. A mastitis infusion system comprising:
a) a syringe barrel;
b) a cannula having a free tip end with an opening thereat;

c) a connecting assembly which connects the cannula to the syringe barrel; and
d) mastitis cannula closure means for covering said cannula, said mastitis cannula closure means including:
i) a substantially rigid cap-like member having a proximal end and a distal end;
ii) restriction means for restricting passage of the free tip end of said cannula out of said cap-like member, said restriction means being connected with the distal end of said cap-like member;
iii) mounting means for removably holding said cap-like member in surrounding relation to said cannula at a first position at which said cap-like member is in covering relation to the free tip end of said cannula, said mounting means permitting removal of said cap-like member from said surrounding relation to expose at least substantially the entire length of said cannula; and
iv) said substantially rigid portion of said cap-like member being movable by an external force from said first position to a second position at which said mounting means removably holds said cap-like member in surrounding relation to said cannula and at which said free tip end extends through said restriction means and only partially out of said cap-like member, and said substantially rigid portion being maintained at said second position after removal of said external force.

8. A mastitis infusion system according to Claim 7, wherein said connecting assembly includes first and second axially spaced grooves, and said mounting means includes bead means on an inner wall of said cap-like member for engaging within said first groove in said first position and said second groove in said second position.

9. A mastitis infusion system according to Claim 8, wherein said connecting assembly includes a cylindrical member, and said first and second grooves are annular grooves axially spaced along said cylindrical member.

10. A mastitis infusion system comprising:
a) a syringe barrel;
b) a cannula having a free tip end with an opening thereat;
c) a connecting assembly which connects the cannula to the syringe barrel; and
d) mastitis cannula closure means for covering said cannula, said mastitis cannula closure means including:

i) a cap-like member having a proximal end and a distal end, said cap-like member including a substantially rigid portion and a collapsible portion connected with said substantially rigid portion;

ii) restriction means for restricting passage of the free tip end of said cannula out of said cap-like member, said restriction means being connected with the distal end of said cap-like member;

iii) mounting means for removably holding said cap-like member in surrounding relation to said cannula at a first position at which said cap-like member is in covering relation to the free tip end of said cannula, said mounting means permitting removal of said cap-like member from said surrounding relation to expose at least substantially the entire length of said cannula; and

iv) said collapsible portion being collapsible in an axial direction of said cannula by an external axial force so as to move said substantially rigid portion of said cap-like member from said first position to a second position at which said free tip end extends through said restriction means and only partially out of said cap-like member, and said collapsible portion being maintained in said collapsed condition and said substantially rigid portion being maintained at said second position after removal of said external force.

**11.** A mastitis infusion system according to Claim 10, wherein said connecting assembly includes a groove, and said mounting means includes inturned lip means connected with said cap-like member for engaging within said groove.

**12.** A mastitis infusion system according to Claim 11, wherein said groove is an annular groove and said inturned lip means extends radially inward from the proximal end of said cap-like member.

# FIG.1

# FIG.2

# FIG.6

# FIG.7

FIG.3

FIG.4

FIG.5

EP 0 510 575 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,Y | US-A-4 850 970 (SUTHERLAND) 25 July 1989<br><br>* the whole document *<br>--- | 1,2,5,<br>7-11 | A61D1/02<br>A61M5/32 |
| Y | US-A-4 900 311 (STERN) 13 February 1990 | 1,2,7,8,<br>10,11 | |
| A | * column 3, line 23 - column 4, line 27; figure 2 *<br>--- | 5,6 | |
| Y | US-A-2 571 653 (BASTIEN) 16 October 1951 | 5,7-9 | |
| A | * the whole document *<br>--- | 10-12 | |
| A | US-A-3 134 380 (ARMAO) 26 May 1964<br>* the whole document *<br><br>----- | 3 | |
| | | | TECHNICAL FIELDS SEARCHED (Int. Cl.5 )<br><br>A61D<br>A61M |

The present search report has been drawn up for all claims

| Place of search | Date of completion of the search | Examiner |
|---|---|---|
| THE HAGUE | 30 JULY 1992 | VANRUNXT J. |

EPO FORM 1503 03.82 (P0401)